# EUROPEAN PATENT APPLICATION

(11) **EP 4 137 503 A1**
(43) Date of publication of application: **22.02.2023**
(21) Application number: 21192338.8
(22) Date of filing: 20.08.2021
(51) Int. Cl.: C07K 7/06, A61K 38/00, C07K 14/81, C12N 9/50

(54) **NOVEL CATHEPSIN INHIBITORS**

(71) Applicant: École Polytechnique Fédérale de Lausanne (EPFL), 1015 Lausanne (CH)
(72) Inventor: The designation of the inventor has not yet been filed
(74) Representative: Vossius & Partner Patentanwälte Rechtsanwälte mbB

(57) **Abstract**

The present invention relates to a cathepsin inhibitor comprising or consisting of Formula (I)

(X₁)(X₂)(X₃)(X₄)(Y)(X₅)(X₆)(X₇) Formula (I)

wherein (X₁) is an amino acid selected from K, L, F and M; (X₂) is an amino acid selected from L, A, D, E, F, G, H, I, K, M, N, S, W, Y, Q and R; (X₃) is an amino acid selected from R, A and L; (X₄) is an amino acid selected from M, I, K, V and Y; (X₅) is an amino acid selected from P, A, I, L, V, Wand Y; (X₆) is an amino acid selected from K, A, E, F, G, I, L, M, N, Q, S, T, V, W, Y and Q; (X₇) is an amino acid selected from D, A, E, F, G, I, L, M, N, P, T, V, W, Y, Q and R; and (Y) is a Michael acceptor.

## Description

The present invention relates to a cathepsin inhibitor comprising or consisting of Formula (I)

(X₁)(X₂)(X₃)(X₄)(Y)(X₅)(X₆)(X₇) Formula (I)

wherein (X₁) is an amino acid selected from K, L, F and M; (X₂) is an amino acid selected from L, A, D, E, F, G, H, I, K, M, N, S, W, Y, Q and R; (X₃) is an amino acid selected from R, A and L; (X₄) is an amino acid selected from M, I, K, V and Y; (X₅) is an amino acid selected from P, A, I, L, V, Wand Y; (X₆) is an amino acid selected from K, A, E, F, G, I, L, M, N, Q, S, T, V, W, Y and Q; (X₇) is an amino acid selected from D, A, E, F, G, I, L, M, N, P, T, V, W, Y, Q and R; and (Y) is a Michael acceptor.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

In the past years, functional annotations of cancer genomic lesions have steered cancer treatment towards more targeted and personalized therapies. This approach in combination with the introduction of immunotherapies has significantly improved cancer treatment. However, curative approaches are still rare and resistance to treatments and disease relapse are frequent and linked with tumor evolution.

Recently cathepsin proteins and in particular cathepsin S were identified as an interesting therapeutic target in cancer and immunological diseases (see for example, Wilkinson et al. (2016), Biological Chemistry https://doi.org/10.1515/hsz-2015-0114).

Dheilly et al. (2020), Cancer Cell, doi: 10.1016/j.ccell.2020.03.016 analyzed genomic data from several cohorts of follicular lymphoma (FL) patients and observed that 5.7% of FL patients harbor Y132D hot-spot mutation of cathepsin S. They demonstrated that somatic alteration Y132D is a gain-of-function mutation that enhances the protein activity. It was observed that cathepsin proteins are also overexpressed in several tumor types and furthermore shown that cathepsin S mutation and over-expression accelerate tumorigenesis in animal models of lymphoma. Next, the genetic knock-out of cathepsin S demonstrated that loss of its activity reduces tumorigenesis and improves response to immunotherapies in pre-clinical studies.

Previous studies have shown that cathepsin proteins are potential therapeutic targets to block tumor metastasis. Cathepsin B (CTSB) for instance is frequently overexpressed in human breast cancer and correlates with a poor prognosis. Inhibition of CTSB attenuates metastasis and tumor growth in mouse models of human cancers.

In general, cathepsin proteins are important for the regulation of antigen presentation and adaptive immune response, and inhibition of cathepsin proteins could be beneficial in genetic auto-immune diseases.

Hence, in order to further advance the therapeutic options for treating and preventing cancer and immunological diseases cathepsin inhibitors are needed. Such cathepsin inhibitors can be used alone or in combination with immunotherapies for the treatment and the prevention of cancer and immunological diseases.

Thus, the present invention relates in a first aspect to a cathepsin inhibitor comprising or consisting of Formula (I)

(X₁)(X₂)(X₃)(X₄)(Y)(X₅)(X₆)(X₇) Formula (I)

wherein (X₁) is an amino acid selected from K, L, F and M; (X₂) is an amino acid selected from L, A, D, E, F, G, H, I, K, M, N, S, W, Y, Q and R; (X₃) is an amino acid selected from R, A and L; (X₄) is an amino acid selected from M, I, K, V and Y; (X₅) is an amino acid selected from P, A, I, L, V, Wand Y; (X₆) is an amino acid selected from K, A, E, F, G, I, L, M, N, Q, S, T, V, W, Y and Q; (X₇) is an amino acid selected from D, A, E, F, G, I, L, M, N, P, T, V, W, Y, Q and R; and (Y) is a Michael acceptor.

Cathepsins are protease enzymes, categorized into multiple families. They can be a serine protease, cysteine protease, or aspartyl protease. There are currently about 15 classes of cathepsins in humans (Patel et a. (2018), Biomed Pharmacother. 2018 Sep; 105: 526-532). These enzymes are active in the low pH milieu of lysosomes and are versatile in their functions. Like other enzymes, they are vital for the normal physiological functions such as digestion, blood coagulation, bone resorption, ion channel activity, innate immunity, complement activation, apoptosis, vesicular trafficking, autophagy, angiogenesis, and proliferation among scores of others. Numerous pathologies have been attributed to the dysregulated cathepsins, some of which include arthritis, periodontitis, pancreatitis, macular degeneration, muscular dystrophy, atherosclerosis, obesity, stroke, Alzheimer's disease, schizophrenia, tuberculosis, and Ebola.

Cathepsin S and B are both cysteine proteases and known to be involved in cancer. Cathepsin S (CTSS) belongs the family of lysosomal cysteine proteases. In macrophages and dendritic cells, it functions as a major endoproteinase that cleaves the invariant chain (CD74) from the major histocompatibility complex (MHC) class II prior to antigen presentation. In addition to its role in antigen processing, CTSS can remodel the extracellular matrix in various tissues when secreted. Unlike other acidic lysosomal proteases, CTSS remains catalytically active and stable in neutral pH, allowing for its proteolytic activity when secreted (Tjondrokoesoemo et al (2016), J. Biol. Chem. 291: 9920-9928, 2016).

Cathepsin B is a member of the papain-like family of cysteine proteases and is synthesized as a preproenzyme of 339 amino acids with a calculated molecular weight of approximately 38 kDa. Cathepsin B is a bilobal protein with its catalytic site located at the interface between the two lobes. The amino acids cysteine, histidine and aspartic acid comprise the catalytic triad of the enzyme, with cysteine on the left lobe interacting with histidine on the right lobe to catalyze peptide bond cleavage. Cathepsin B exhibits both endopeptidase and exopeptidase activity (Cavallo-Medved et a. (2011), AFCS Nat Mol Pages. 2011; A000508).

The term "cathepsin inhibitor" refers to agents being able to inhibit the protease activity of cathepsin, in particular cathepsin S or B. Inhibition of Cathepsin S or B can be assessed for example by measuring the enzymatic transformation of Cathepsin S or B substrates (peptides of 9-10 amino acids) into products (peptides of 4-6 amino acids), for example, by a FRET assay as described in the examples. Inhibition of Cathepsin S or B can be also assessed at the functional level, for example, by testing the inhibitory effect by measuring changes of antigen repertoire, T cell activation and in the case of cathepsin S by the accumulation of CD74(p10) protein.

The cathepsin inhibitor comprises the 7 amino acids (X₁) to (X₇) and therefore may comprise further amino acids at the "N-terminus" (i.e. next to (X₁)) and/or C-terminus (i.e. next to (X₇)). The total number of amino acids in the cathepsin inhibitor is with increasing preference 50 or less, 40 or less, 30 or less, 20 or less, 15 or less, 10 or less, 9 or less, 8 or less and most preferably 7. In the most preferred case no amino acids other than (X₁) to (X₇) are present in the cathepsin inhibitor.

The cathepsin inhibitor might be linear of circular and is preferably linear. In case of a circular cathepsin inhibitor the circle is preferably formed by connecting the side chains of two amino acids either directly (e.g. via disulfide bonds in the case of two cysteine) or via a connecting molecule. It is in particular to form a circle between the side chains of (X₁) and (X₇) or two amino acids that can be found at the "N-terminus" (i.e. next to (X₁)) and C-terminus (i.e. next to (X₇)) of the cathepsin inhibitor.

The term "connecting molecule" as used herein refers to a molecule which is capable of connecting the side chains of at least two amino acids via covalent bonds whereby a ring or cycle is formed. A covalent bond is a chemical bond that involves the sharing of electron pairs between atoms. For this purpose the connecting molecule comprises at least two functional groups. A functional group designates a specific group of atoms or bonds within the connecting molecule that is responsible for connecting the side chain of at least two amino acids to the connecting molecule. For this purpose the functional group in general undergoes a chemical reaction with at least one atom in the side chain of the amino acid. The chemical reaction results in a covalent bond between the functional group and the side chain. Non-limiting examples of functional groups are alkyl, alkenyl, alkynyl, phenyl, benzyl, halo (such as fluoro or chloro), hydroxyl, carbonyl, aldehyde, haloformyl, carbonate ester, carboxylate, carboxyl, ester, methoxy, hydroperoxy, peroxy, ether, hemiacetal, hemiketal, acetal, ketal, orthoester, heterocycle, orthocarbonate ester, carboxamide, amine (including primary, secondary and tertiary amine), imine, azide, azo compound, (iso)cyanate, nitrate, nitrite, sufhydryl, sulfide, disulfide, sulfinyl, sulfonyl, sulfin, sulfo, (iso)thiocyanate, carbonothiol, carbonothioyl, phosphino (such as phospate), borono, boronate, boroniate and borino. The at least two functional groups of the connecting molecule may be the same or different and are preferably the same.

The term "amino acid" as used herein refers to an organic compound composed of amine (-NH₂ or -NH) and carboxylic acid (-COOH) functional groups, generally along with a side-chain specific to each amino acid. The simplest amino acid glycine does not have a side chain (formula H₂NCH₂COOH). In amino acids that have a carbon chain attached to the α-carbon (such as lysine), the carbons are labelled in the order α, β, γ, δ, and so on. In some amino acids, the amine group may be attached, for instance, to the α-, β- or y-carbon, and these are therefore referred to as α-, β- or γ-amino acids, respectively. The term "amino acid" preferably describes α-amino acids (also designated 2-, or alpha-amino acids) which generally have the generic formula H₂NCHRCOOH, wherein R is an organic substituent being designated "side-chain", and also beta-, gamma- and delta-amino acids, that contain multiple carbon atoms between the amine and the carboxylic acid. In the simplest α-amino acid alanine (formula: H₂NCHCH₃COOH) the side chain is a methyl group. The amino acids are in accordance with the present invention are L-amino acids or D-amino acids.

The amino acids comprise the so-called standard or canonical amino acids. These 21 α-amino acids are encoded directly by the codons of the universal genetic code. They are the proteinogenic α-amino acids found in eukaryotes. These amino acids are referred to herein in the so-called one-letter code:

| | | | |
|---|---|---|---|
| **G** | Glycine | **P** | Proline |
| **A** | Alanine | **V** | Valine |
| **L** | Leucine | **I** | Isoleucine |
| **M** | Methionine | **C** | Cysteine |
| **F** | Phenylalanine | **Y** | Tyrosine |
| **W** | Tryptophan | **H** | Histidine |
| **K** | Lysine | **R** | Arginine |
| **Q** | Glutamine | **N** | Asparagine |
| **E** | Glutamic Acid | **D** | Aspartic Acid |
| **S** | Serine | **T** | Threonine |
| **U** | Selenocysteine | | |

The four amino acids (X₁)(X₂)(X₃)(X₄) as well as the three amino acids (X₅)(X₆)(X₇) within the inhibitor of the invention are each connected to form a "peptide". A peptide generally designates a short chain of amino acids linked by peptide bonds. The term "peptide" as used herein, thus, designates short chains of amino acids that are preferably linked by peptide bonds.

The cathepsin inhibitor of the invention is not only composed of amino acids being connected to form a peptide but comprises in addition (Y), which is a Michael acceptor. Michael additions (or Michael reactions) are C-C bond formation reactions in which nucleophilic addition of a carbanion or another nucleophile to an α,β-unsaturated carbonyl compound containing an electron withdrawing group occurs. In this scheme, R and R' on the nucleophile (the Michael donor) represent electron-withdrawing substituents such as acyl and cyano groups, which make the adjacent methylene hydrogen sufficiently acidic to form a carbanion when reacted with the base, B: as shown in the below reaction. The R" substituent is bound to the activated alkene, also called a Michael acceptor. The nature of the Michael acceptor will be further detailed herein below.

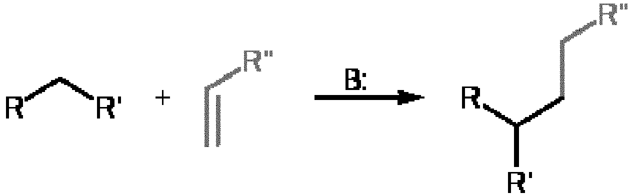

Since the cathepsin inhibitor of the invention is composed of seven amino acids and the Michael acceptor as illustrated by formula (I) it is to be understood that the cathepsin inhibitor of the invention does not occur in nature but has to be chemically synthesized as also illustrated by the appended examples. The cathepsin inhibitor also cannot be encoded by a nucleic acid molecule, such as an expression vector.

As can be taken from the appended examples, the cathepsin inhibitor of the invention was designed based on the knowledge that CD74 is a natural substrate of cathepsin S and the computational analysis of the interaction site between CD74 and cathepsin S. After the amino acids of CD74 that interact with cathepsin S were identified several candidate cathepsin inhibitors were produced by adding a Michael acceptor at different position. The position of (Y) within formula (I) was identified as the ideal position regarding the inhibition of cathepsin S. These efforts led to the identification of the very potent non-natural peptide cathepsin S inhibitor P3. It is important to note that both the amino acids and the Michael acceptor contribute to the inhibitory power of the cathepsin inhibitor of the invention.

In addition, mutagenesis screens were performed in order to further increase the inhibitory capacity of the cathepsin inhibitor P3 of the invention. By these measures finally the non-natural peptide inhibitor C10 was identified as a very potent cathepsin S inhibitor. By a further mutagenesis screen the specificity of the cathepsin inhibitor could be changed from cathepsin S to cathepsin B. The screen resulted in the very potent cathepsin B inhibitor D5.

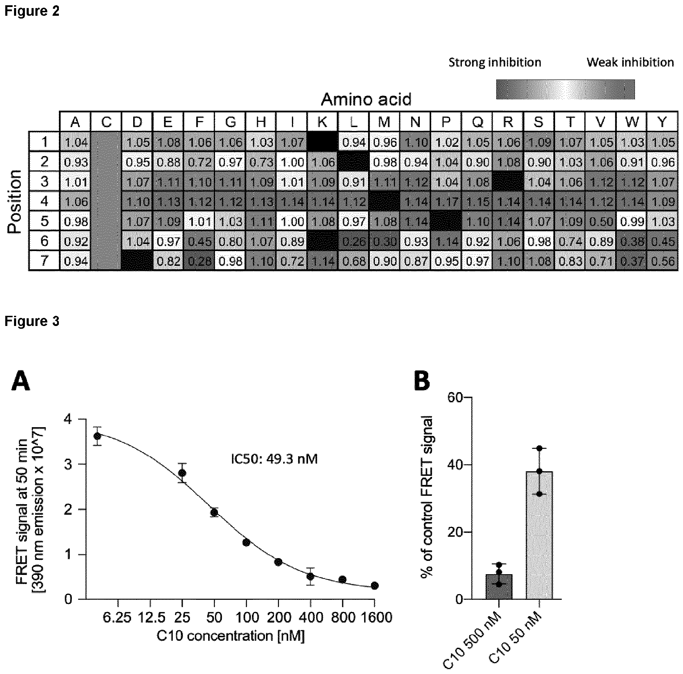

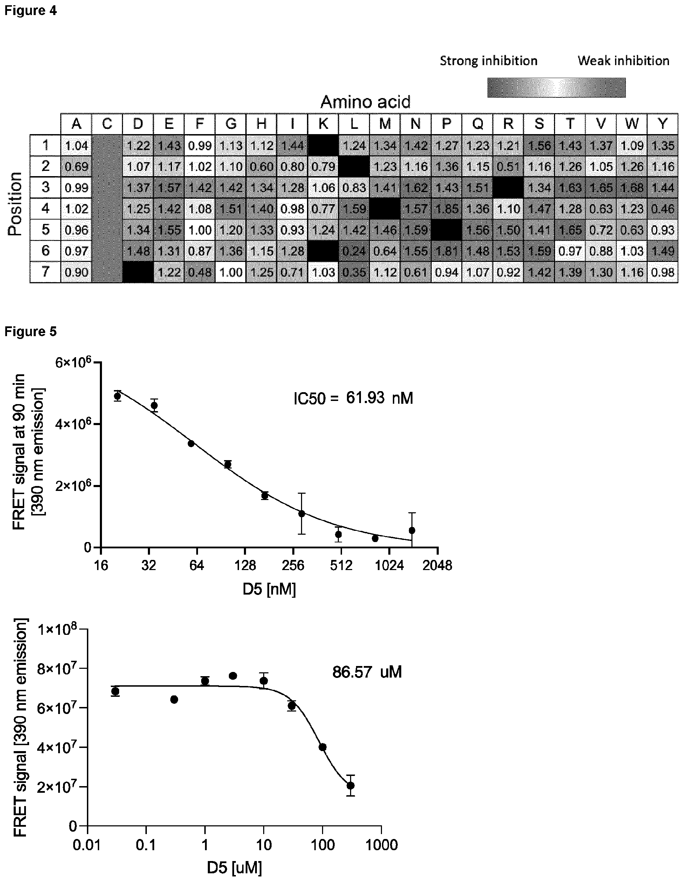

The results of the mutagenesis screens that lead to the inhibitors C10 and D5 are shown in Figures 2 and 4. It can be taken from these figures that the screens are based on single amino acid substitutions in the initially identified cathepsin inhibitor P3. All amino acid substitutions that had no effect on the inhibitory capacity of P3 or even resulted in an improvement of the inhibitory capacity of P3 vis-à-vis cathepsin S (Figure 2) or cathepsin B (Figure 4) have a value of 1.0 and below.

The amino acids of P3 along with the amino acid substitutions of Figures 2 and 4 that had no effect or resulted in an improvement together gave raise to formula (I). The structure of formula (I) is therefore a Markush formula of very potent cathepsin inhibitors, in particular very potent cathepsin S and/or B inhibitors. To the best knowledge of the inventors the structure of formula (I), let alone its cathepsin inhibitory capacity is not known from the prior art.

The preferred amino acids in the structure of formula (I) are those amino acids that further improved the inhibitory capacity of P3. A combination of such amino acids in one cathepsin inhibitor resulted in the inhibitors C10 and D5.

As far as the amino acid position (X₇) is W it is of note that W resulted in an improvement regarding the inhibition of cathepsin S, while it slightly diminishes the inhibition of cathepsin B. However, W at the amino acid position (X₇) is also advantageous in connection with the inhibition of cathepsin B since it was found that W at the amino acid position (X₇) generally improves the solubility of the cathepsin inhibitor of the invention.

In accordance with a preferred embodiment of the first aspect of the invention the cathepsin inhibitor selectively inhibits cathepsin S and comprises or consists of Formula (II)

(X₁)(X₂)(X₃)(X₄)(Y)(X₅)(X₆)(X₇) Formula (II)

wherein (X₁) is an amino acid selected from K, L, M, and is preferably K; (X₂) is an amino acid selected from L, A, D, E, F, G, H, I, M, N, Q, S, W, Y, and is preferably F or H; (X₃) is an amino acid selected from R and L; (X₄) is the amino acid M; (X₅) is an amino acid selected from P, A, I, L, V, W, and is preferably V; (X₆) is an amino acid selected from K, A, E, F, G, I, L, M, N, Q, S, T, V, W, Y, and is preferably selected from F, L, M, W and Y, and is most preferably L or M; (X₇) is an amino acid selected from D, A, E, F, G, I, L, M, N, P, Q, T, V, W, Y, and is preferably selected from F, I, L, V, W and Y and is most preferably F or W.

Formula (II) is based on the initially identified cathepsin inhibitor P3 and the mutagenesis screen for even more effective cathepsin S inhibitors as shown in Figure 2. All amino acid substitutions that had no effect on the inhibitory capacity of P3 or even resulted in an improvement of the inhibitory capacity of P3 vis-à-viscathepsin S have a value of 1.0 and below. These amino acids and the amino acids of the cathepsin inhibitor P3 form the basis for the definition of (X₁) to (X₇) in formula (II). The preferred amino acids for (X₁) to (X₇) in the structure of formula (II) are those amino acids that further improved the inhibitory capacity of P3 vis-à-vis cathepsin S.

In accordance with a more preferred embodiment of the first aspect of the invention the cathepsin inhibitor comprises or consists of KLRM(Y)PKD or KHRM(Y)VMW and preferably comprises or consists of KHRM(Y)VMW.

The inhibitor KLRM(Y)PKD is the inhibitor P3 and the inhibitor KHRM(Y)VMW is the inhibitor C10, wherein (Y) is the Michael acceptor. The inhibitor C10 is most preferred since it is an optimized version of the inhibitor of P3 harbouring selected amino acid substitutions that were found to further increase the inhibitory capacity of P3 vis-à-viscathepsin S.

In accordance with another preferred embodiment of the first aspect of the invention the cathepsin inhibitor selectively inhibits cathepsin B and comprises or consists of Formula (III)

(X₁)(X₂)(X₃)(X₄)(Y)(X₅)(X₆)(X₇) Formula (III)

wherein (X₁) is the amino acid K or F; (X₂) is an amino acid selected from A, H, I, K and R, and is preferably H or R; (X₃) is an amino acid selected from A, R and L, and is preferably L; (X₄) is an amino acid selected from I, K, V and Y, and is preferably K or Y; (X₅) is an amino acid selected from A, I, Y, V and W, and is preferably V or W; (X₆) is an amino acid selected from A, F, L, M, T and V, and is preferably L or M; (X₇) is an amino acid selected from A, F, I, L, N, P, R, W and Y and is preferably F or L.

Formula (III) is based on the mutagenesis screen based on the cathepsin S inhibitor P3 in order to identify cathepsin B inhibitors as shown in Figure 4. All amino acid substitutions that resulted in an improvement of the inhibitory capacity of P3 vis-à-vis cathepsin B have a value of below 1.0. These amino acids form the basis for the definition of (X₁) to (X₇) in formula (III). The preferred amino acids for (X₁) to (X₇) in the structure of formula (III) are those amino acids that most effectively confer inhibitory activity vis-à-vis cathepsin B, noting that W at position is a preferred option of (X₇) since it improves the solubility of the inhibitor.

In accordance with a more preferred embodiment of the first aspect of the invention the cathepsin inhibitor comprises or consists of KRRY(Y)WMW.

The inhibitor KRRY(Y)WMW is the inhibitor D5, wherein (Y) is the Michael acceptor. The inhibitor D5 is the most preferred cathepsin B inhibitor since it harbours selected amino acid substitutions that were found to increase the inhibitory capacity of P3 vis-à-vis cathepsin B.

In accordance with a further preferred embodiment of the first aspect of the invention the Michael acceptor is an enone, a nitro group or a sulfonyl fluoride and is preferably an enone.

The Michael acceptor is usually a ketone, which makes the compound an enone, but it can also be a nitro group or a sulfonyl fluoride. An enone is a type of an α,β-unsaturated carbonyl that consists of an alkene conjugated to a ketone. The simplest enone is methyl vinyl ketone (butenone, CH2=CHCOCH3). Enones are typically produced using an aldol condensation or Knoevenagel condensation. Some commercially significant enones produced by condensations of acetone are mesityl oxide (dimer of acetone) and phorone and isophorone (trimers).

In accordance with a further preferred embodiment of the first aspect of the invention the Michael acceptor has the general formula (IV) or (V) 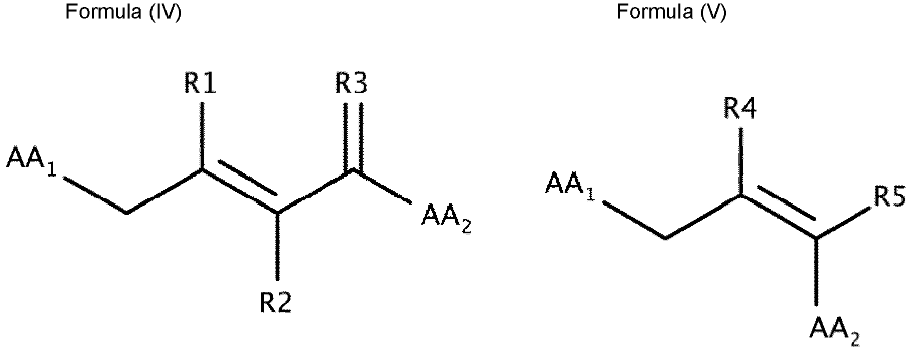 wherein
R1, R2 and R4 each independently is H or a halogen, wherein the halogen is preferably F;
R3 is chalcogen and is preferably O or S; and

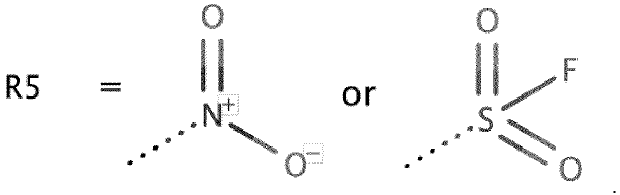

The compound of formula (IV) is an enone. The compound of formula (V) according to the first option of R5 is a nitro group, and the compound of formula (V) according to the second option of R5 is a sulfonyl fluoride. The enone is again most preferred.

The halogen is preferably selected from fluorine (F), chlorine (CI), bromine (Br) and iodine (I), more preferably from F, Cl and Br, even more preferably from F and Cl and is most preferably F.

A chalcogen is a chemical element in group 16 of the periodic table. The halogen is preferably selected from oxygen (O), sulfur (S), selenium (Se) and tellurium (Te), more preferably from O, S and Se, even more preferably from O and S and is most preferably O.

In accordance with a preferred embodiment of the first aspect of the invention the cathepsin inhibitor inhibits cathepsin S or B with IC₅₀ of below 200 µM, preferably below 100µM and most preferably below 75µM.

The above IC₅₀ values of the cathepsin inhibitor can be determined by art-established techniques and are preferably determined by the FRET assay as described in the examples herein below. Details on the assay can be found in particular in the section "specificity assay" of the examples.

The IC₅₀ values of the cathepsin inhibitor of the invention is with increasing preference below 50µM, below 25µM below 10µM, below 1µM, below 500nM, below 250nM, below 200nM, and below 100nM. In this respect it is of note that the cathepsin S inhibitor C10 displays an IC₅₀ of 49.3 nM in the FRET assay of the appended examples (Figure 3) and the cathepsin B inhibitor D5 displays an IC₅₀ of 61.93 nM in the FRET assay of the appended examples (Figure 5).

In accordance with another preferred embodiment of the first aspect of the invention the cathepsin inhibitor comprises at its N- or C-terminus, preferably at its C-terminus a linking moiety.

A linking moiety (or linker moiety) generally relates to a functional group that is covalently bound to the inhibitor of the invention and can serve to link, generally covalently link a further compound to the inhibitor of the invention. Examples of such heterologous compounds will be provided herein below.

In accordance with a further preferred embodiment of the first aspect of the invention the cathepsin the linking moiety is an azide, alkyne phenol, secondary or tertiary amine, hydroxyl group, carbamate, or carbonate group, and is preferably an azide.

Non-limiting examples of suitable linking moieties are an azide, alkyne phenol, secondary or tertiary amine, hydroxyl group, carbamate. An azide is preferred since its it used in the examples in order to covalently link the inhibitor of the invention to an antibody.

A linking moiety, such as an alkyne or azide may be used in click-chemistry reactions. "Click-chemistry" is an art-established term; see e.g. Kolb et al. (2001) Click chemistry: diverse chemical function from a few good reactions. Angew. Chem. Int. Ed. 40 (11):2004; Sletten et al. (2009) Bioorthogonal Chemistry: Fishing for Selectivity in a Sea of Functionality. Angew. Chem. Int. Ed.48:6998; Jewett et al.(2010) Cu-free click cycloaddition reactions in chemical biology. Chem. Soc. Rev. 39(4):1272; Best et al. (2009) Click Chemistry and Bioorthogonal Reactions: Unprecedented Selectivity in the Labeling of Biological Molecules. Biochemistry.48:6571; and Lallana et al. (2011) Reliable and Efficient Procedures for the Conjugation of Biomolecules through Huisgen Azide-Alkyne Cycloadditions. Angew. Chem. Int. Ed. 50:8794. While there are a number of reactions that fulfill the criteria, the Huisgen 1,3-dipolar cycloaddition of azides and terminal alkynes has emerged as the frontrunner and are also preferred in connection with the present invention.

The present invention relates in a second aspect to a fusion construct, wherein the cathepsin inhibitor of the first aspect is fused to a heterologous compound, preferably fused to a pharmaceutically and/or diagnostically active compound.

The definitions and preferred embodiments of the first aspect of the invention apply *mutatis mutandis* to the second aspect of the invention as far as being amenable with this second aspect.

A "fusion construct" has used herein defines the fusion of the inhibitor of the invention to a heterologous compound. The heterologous compound is not particularly limited. It can be a proteinaceous compound of a non-proteinaceous compound. Examples of a proteinaceous and non-proteinaceous compounds will be provided herein below. The compound may be selected from the group consisting of a pharmaceutically active compound and/or a diagnostically active compound. A pharmaceutically active compound is capable of exerting a curative or disease preventive effect *in vivo* within a subject and a diagnostically active compound is useful for diagnosing a disease *in vivo* within a subject or *in vitro* within a sample obtained from the subject.

In the case the compound is a proteinaceous compound (e.g. a cytokine or chemokine as described herein below), the fusion construct is also a fusion protein as defined herein above. In other words, the term "fusion constructs" comprises fusion proteins. The compound may either be directly fused to the inhibitor or via a linker. The linker is preferably selected from the group consisting of alkyl with 1 to 30 carbon atoms, polyethylene glycol with 1 to 20 ethylene moieties, polyalanine with 1 to 20 residues, caproic acid, substituted or unsubstituted poly-p-phenylene and triazol.

In a further preferred embodiment, said linker is selected from the group consisting of amino-n-alkyl, mercapto-n-alkyl, amino-n-alkyl-X-alkyl, mercapto-n-alkyl-X-alkyl, wherein X is selected from the group consisting of O, S-S and SO₂ and wherein the alkyl groups independently from each other have from 1 to 30 carbon atoms, preferably 3, 6 or 12 carbon atoms; or oligoethylenglycols having from one to about ten ethylenglycol moieties, preferably tri- or hexa-ethylenglycol. These and further suitable linkers are well known in the art and commercially available (see, for example, the catalogue from Glen Research, 22825 Davis Drive, Sterling, Virginia, 20164 USA). Further examples of linkers are the following: 5'-amino-modifiers (see e.g. B.A. Connolly and P. Rider, Nucleic Acids Res., 1985, 13, 4485; B.S. Sproat, B.S. Beijer, P. Rider, and P. Neuner, Nucleic Acids Res., 1987, 15, 4837; R. Zuckerman, D. Corey, and P. Shultz, Nucleic Acids Res., 1987, 15, 5305; P. Li, et al., Nucleic Acids Res., 1987, 15, 5275; G.B. Dreyerand P.B. Dervan, Proc. Natl. Acad. Sci. USA, 1985, 82, 968.); 5'-thiol-modifier C6 (see e.g. B.A. Connolly and P. Rider, Nucleic Acids Res., 1985, 13, 4485; B.S. Sproat, B.S. Beijer, P. Rider, and P. Neuner, Nucleic Acids Res., 1987, 15, 4837; R. Zuckerman, D. Corey, and P. Shultz, Nucleic Acids Res., 1987, 15, 5305; P. Li, et al., Nucleic Acids Res., 1987, 15, 5275.); 5'-thiol-modifier C6 S-S and thiol group at the 3'-terminus (see e.g. B.A. Connolly and R. Rider, Nucleic Acids Res., 1985, 13, 4485; B.A. Connolly, NucleicAcids Res., 1987, 15, 3131-3139; N.D. Sinha and R.M. Cook, Nucleic Acids Res., 1988, 16, 2659; A. Kumar, S. Advani, H. Dawar, and G.P. Talwar, Nucleic Acids Res., 1991, 19, 4561; R. Zuckermann, D. Corey, and P. Schultz, Nucleic Acids Res., 1987, 15, 5305; K.C. Gupta, P. Sharma, S. Sathyanarayana, and P. Kumar, Tetrahedron Lett., 1990, 31, 2471-2474; U. Asseline, E. Bonfils, R. Kurfurst, M. Chassignol, V. Roig, and N.T. Thuong, Tetrahedron, 1992, 48, 1233-1254; Gregg Morin, Geron Corporation, Personal Communication.); spacer C3, spacer C12, and dspacer phosphoramidites (see e.g. M. Durard, K. Chevrie, M. Chassignol, N.T. Thuong, and J.C. Maurizot, Nucleic Acids Res., 1990, 18, 6353; M. Salunkhe, T.F. Wu, and R.L. Letsinger, J. Amer. Chem. Soc., 1992, 114, 8768-8772; N.G. Dolinnaya, M. Blumenfeld, I.N. Merenkova, T.S. Oretskaya, N.F.Krynetskaya, M.G. Ivanovskaya, M. Vasseur, and Z.A. Shabarova, Nucleic Acids Res., 1993, 21, 5403-5407; M. Takeshita, C.N. Chang, F. Johnson, S. Will, and A.P. Grollman, J. Biol. Chem., 1987, 262, 10171-10179; M.W. Kalnik, C.N. Chang, A.P. Grollman, and D.J. Patel, Biochemistry, 1988, 27, 924-931.); 3'-amino-modifier C7 CPG (see e.g. J.G. Zendegui, K.M. Vasquez, J.H. Tinsley, D.J. Kessler, and M.E. Hogan, Nucleic Acids Res., 1992, 20, 307.); 3'-Amino Photolabile C6 CPG (see e.g. D.J. Yoo and M.M. Greenberg, J. Org. Chem., 1995, 60, 3358-3364.; H. Venkatesan and M.M. Greenberg, J. Org. Chem., 1996, 61, 525-529; D.L. McMinn and M.M. Greenberg, Tetrahedron, 1996, 52, 3827-3840.

As discussed, in case the heterologous compound is a proteinaceous compound the fusion construct may also be designated "fusion protein". A "fusion protein" according to the present invention contains at least one additional heterologous amino acid sequence apart from the inhibitor of the invention. Often, but not necessarily, these additional sequences will be located at the N- or C-terminal end of the inhibitor of the invention. The amino acid sequence can either be directly or indirectly fused to the inhibitor of the invention. In case of an indirect fusion generally a peptide linker may be used for the fusion, such that a GS₍ₙ₎-linker, wherein n is 1 to 3.

Most preferably the inhibitor of the invention and the heterologous compound are fused via a linking moiety as described herein above.

In accordance with preferred embodiment of the second aspect of the invention the heterologous compound is selected from the group consisting of:
(i) antibody or fragment thereof;
(ii) an antibody mimetic, preferably selected from the group consisting of Anticalins, Affibodies, Adnectins, DARPins, Avimers, Nanofitins, Affilinss, β-Wrapins, ADAPT, Monobodoes, Raslns, FingRs, Pronectins, Centyrins, Affimers, Adhirons, Affitins, αReps, Repebodies, i-bodies, Fynomers and Kunitz domain proteins;
(ii) a cytokine, preferably cytokines selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1alpha and IL-1beta;
(iii) a toxic compound, preferably a small organic compound or polypeptide, and preferably a toxic compound selected from the group consisting of calicheamicin, neocarzinostatin, esperamicin, dynemicin, kedarcidin, maduropeptin, doxorubicin, daunorubicin, auristatin, Ricin-A chain, modeccin, truncated Pseudomonas exotoxin A, diphtheria toxin and recombinant gelonin;
(iv) a chemokine, preferably a chemokine selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, Eotaxin, Eotaxin-2, I-309, MPIF-1, 6Ckine, CTACK, MEC, Lymphotactin and Fractalkine;
(v) a fluorescent dye, preferably a component selected from a Alexa Fluor or Cy dye;
(vi) a photosensitizer, preferably bis(triethanolamine)Sn(IV) chlorine6 (SnChe6);
(vii) a pro-coagulant factor, preferably a tissue factor;
(viii) an enzyme, preferably an enzyme selected from the group consisting of carboxy-peptidases, glucuronidases and glucosidases;
(ix) a radionuclide selected either from the group of gamma-emitting isotopes, preferably 99mTc, 1231, 111In, or from the group of positron emitters, preferably 18F, 64Cu, 68Ga, 86Y, 1241, or from the group of beta-emitters, preferably 1311, 90Y, 177Lu, 67Cu, or from the group of alpha-emitters, preferably 213Bi, 211At; and
(x) nanoparticles.

Among this list of fusion partners (i) an antibody or fragment thereof and (ii) an antibody mimetic are preferred and an antibody or fragment thereof is most preferred. These fusion partners are preferred since they can be designed to bind virtually any target of interest. This includes targets that are of diagnostic and therapeutic interest.

The term "antibody", also known as an immunoglobulin (lg), as used in accordance with the present invention comprises, for example, polyclonal or monoclonal antibodies. Furthermore, comprised in the term "antibody" are multimeric formats, such as minibodies, diabodies, tribodies or triplebodies, or tetrabodies (see, for example, Harlow and Lane "Antibodies, A Laboratory Manual", Cold Spring Harbor Laboratory Press, 1998; Harlow and Lane "Using Antibodies: A Laboratory Manual" Cold Spring Harbor Laboratory Press, 1999; AltshulerEP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. Non-limiting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847). The term "antibody" also includes embodiments such as chimeric (human constant domain, non-human variable domain), single chain and humanised (human antibody with the exception of non-human CDRs) antibodies.

In accordance with the present invention, antibody fragments comprise, *inter alia,* Fab or Fab'fragments, F(ab')₂, Fv or scFvfragments, single domain VH, VL or V-like domains, such as VhH or V-NAR-domains, as well as multimeric formats such as minibodies, diabodies, tribodies, triplebodies, tetrabodies or chemically conjugated Fab'-multimers (see, for example, Altshuler, E. et al. [2010] Biochem. (Mosc.) 75:1584-1605 or Holliger, P. & Hudson, P.J. [2005] Nat. Biotechnol. 23:1126-1136).

"Anticalins" are an emerging class of clinical-stage biopharmaceuticals with high potential as an alternative to antibodies. Anticalin molecules are generated by combinatorial design from natural lipocalins, which are abundant plasma proteins in humans, and reveal a simple, compact fold dominated by a central β-barrel, supporting four structurally variable loops that form a binding site. Reshaping of this loop region results in Anticalin proteins that can recognize and tightly bind a wide range of medically relevant targets, from small molecules to peptides and proteins, as validated by X-ray structural analysis. Their robust format allows for modification in several ways, both as fusion proteins and by chemical conjugation, for example, to tune plasma half-life (Rothe and Skerra (2018) BioDrugs 32, 233-243.).

"Affibodies", in accordance with the present invention, are a family of antibody mimetics derived from the Z-domain of staphylococcal protein A. Affibodies are structurally based on a three-helix bundle domain. An affibody has a molecular mass of around 6 kDa and is stable at high temperatures and under acidic or alkaline conditions. Target specificity is obtained by randomisation of amino acids located in two alpha-helices involved in the binding activity of the parent protein domain (Feldwisch, J & Tolmachev, V. [2012] Methods Mol. Biol. 899:103-126).

"Adnectins" and also "Monobodies", in accordance with the present invention, are based on the 10th extracellular domain of human fibronectin III (10Fn3), which adopts an Ig-like sandwich fold with 2 to 3 exposed loops, but lacks the central disulphide bridge (Gebauer, M. & Skerra, A. [2009] Curr. Opin. Chem. Biol. 13:245-255). Adnectins and Monobodies with the desired target specificity can be genetically engineered by introducing modifications into specific loops or other surface areas of the protein.

"DARPins", in accordance with the present invention, are designed ankyrin repeat domains that provide a rigid interface arising from typically three repeats corresponding to an artificial consensus sequence, whereby six positions per repeat are randomised. Consequently, DARPins lack structural flexibility (Gebauer, M. & Skerra, A. [2009] Curr. Opin. Chem. Biol. 13:245-255).

The term "Avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with desired binding specificity can be selected, for example, by phage display techniques. The target specificity of the different A-domains contained in an avimer may, but do not have to be identical (Weidle UH, et al., (2013), Cancer Genomics Proteomics; 10(4):155-68).

"Nanofitins" and also an "Affitins" are antibody mimetic proteins that are derived from the DNA binding protein Sac7d of Sulfolobus acidocaldarius. Nanofitins and Affitins usually have a molecular weight of around 7kDa and are designed to specifically bind a target moleculeby randomising the amino acids on the binding surface (Mouratou B, Béhar G, Paillard-Laurance L, Colinet S, Pecorari F., (2012) Methods Mol Biol.; 805:315-31 and Koide et al. 1998, J. Mol. Biol. 284:1141-51).

The term "Affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

As used herein, the term "β-Wrapins" designates affibody protein homodimers with a disulfide bond between the pair of Cys28 residues connecting the two identical monomer subunits, referred to as subunits 1 and 2. The scaffold used in engineering β-wrapins is ZAβ₃, an Aβ-binding affibody protein that not only prohibits the initial aggregation of Aβ monomers into toxic forms, but also dissociates preformed oligomeric aggregates by sequestering and stabilizing a β-hairpin conformation of Aβ monomers (Orr et al. (2018), Computers & Chemical Engineering, 116(4):322-332).

As used herein, the term "ABD-Derived Affinity Proteins (ADAPT)" refers to a class of antibody mimetics that has been created using the albumin-binding domain (ABD) of streptococcal protein G as a stable protein scaffold (Garousi et al (2015), Cancer Res.; 75(20):4364-71). By diversifying a surface of the domain that is not directly involved in albumin binding, molecules can be selected to bind a novel target and still retain their ability to bind albumin. This strategy has been used to select binders to a number of proteins, for example, the cancer-related epidermal growth factor receptor 3.

As used herein "Raslns" are 10Fnlll-based antibody mimetics. Hence, they use the 10th domain of human fibronectin as their scaffold Raslns are disulfide-free intrabodies. They were shown to be stable inside cells and also when fused with a fluorescent protein label (Cetin eat al. (2017), J Mol Biol.; 429(4):562-573).

As used herein, the term "FingRs (Fibronectin intrabodies generated with mRNA display)" designates recombinant antibody-like proteins also being based on the 10Fnlll scaffold (Gross eat al. (2013), Neuron.; 78(6): 971-985.).

As used herein, the term "Pronectins" designates recombinant antibody-like proteins being based on the fourteenth type-III scaffold of human fibronectin (14Fn3). The well-characterized fibronectin protein is prevalent throughout the human body. Human fibronectin, an extracellular protein, is naturally abundant in human serum. Intelligent loop-diversity has been designed to closely mimic the natural human repertoire and avoid sequence immunogenicity. The intrinsic properties of a Pronectin align with the pharmacological properties needed to make it a successful drug, including high potency, specificity, stability, favorable small size, and high-yield production in *E. coli* and yeast (http://www.protelica.com/pronectin_tech.html).

As used herein, the term "Centyrins" designates recombinant antibody-like proteins being based on the consensus tenascin FN3 framework (Tencon) (Diem et al. (2014), Protein Eng., Des. and Sel. 27, 419-429). Centryins against different targets, e.g. human c-MET, rTNFα and mIL-17A, were generated.

As used herein, "Affimers" refer to small proteins that bind to target molecules with similar specificity and affinity to that of antibodies. These engineered non-antibody binding proteins are designed to mimic the molecular recognition characteristics of monoclonal antibodies in different applications. In addition, these affinity reagents have been optimized to increase their stability, make them tolerant to a range of temperatures and pH, reduce their size, and to increase their expression in *E. coli and* mammalian cells. Derived from the cysteine protease inhibitor family of cystatins, which function in nature as cysteine protease inhibitors, these 12-14 kDa proteins share the common tertiary structure of an α-helix lying on top of an anti-parallel β-sheet (Tiede et al. (2017), eLife.; 6: e24903).

The class of recombinant antibody-like proteins designated as "Adhirons" herein is based on a phytocystatin consensus sequence as the scaffold (Tiede et al. (2014) Protein Eng. Des. Sel. 27, 145-55).

The class of recombinant antibody-like proteins designated as "αRep" herein is derived from alpha-helicoidal HEAT-like repeat protein scaffolds. In more detail, The αRep proteins are derived from a natural family of modular proteins comprising alpha-helical repeats, related to HEAT repeats, named after Huntingtin, the elongation factor 3 (EF3), the protein phosphatase 2A (PP2A), and the yeast kinase TOR. The association of several HEAT repeats forms alpha-solenoids of various lengths, which are naturally found in a number of cellular proteins involved in intracellular transport and protein-protein interaction (Hadpech et al. (2017), Scientific Reports; 7:Article number16335).

As used herein, the term "Repebodies" designates recombinant antibody-like proteins which are composed of leucine-rich repeat (LRR) modules. In more detail, the binding scaffold of Repebodies is based on variable lymphocyte receptors, which are nonimmunoglobulin antibodies composed of LRR modules in jawless vertebrates. A template scaffold was first constructed by joining consensus repeat modules between the N- and C-capping motifs of variable lymphocyte receptors. The N-terminal domain of the template scaffold was redesigned based on the internalin-B cap by analyzing the modular similarity between the respective repeat units using a computational approach (Lee at al. (2012), Proc Natl Acad Sci; 109(9): 3299-3304).

As used herein, the term "i-bodies" refers to recombinant antibody-like proteins built on the scaffold of a human protein and engineered with two loops that mimic the shape of shark antibodies. These loops are responsible for binding or interacting with a particular target (in circulation or on a cell) that is causing disease. The i-body is a human analogue of the antigen binding domain of the shark antibody, which combines the advantages of monoclonal antibodies (high target specificity and affinity) with the beneficial stability features of small molecules (https://www.ibodies.eu/).

As used herein, the term "Fynomer" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDa and domains with the required target specificity can be selected by display techniques such as phage display (Weidle et al., (2013), Cancer Genomics Proteomics; 10(4):155-68).

Cytokines, toxic compounds, and chemokines are of interest as fusion partners since they may be useful in the treatment of prevention of a disease, including cancer and immunological disorders. Fluorescent dyes and photosensitizers are useful as diagnostic compounds. Enzymes act as biological catalysts and accelerate chemical reactions. Depending on the nature of the enzyme it can be can of therapeutic or diagnostic value. Also depending on the nature of the radionuclide the radionuclide can be of therapeutic (e.g. 177Lu) or diagnostic value (68Ga).

A nanoparticle or is usually defined as a particle of matter that is between 1 and 100 nanometres (nm) in diameter. Peptide-nanoparticle conjugates (PNCs) have recently emerged as a versatile tool for biomedical applications. Synergism between the two promising classes of materials allows enhanced control over their biological behaviors, overcoming intrinsic limitations of the individual materials (Jeong et al. (2018), Nano Converg. 5:38 and Spicer et al. (2018), Chem Soc Rev.; 47(10):3574-3620). While the inhibitor of the invention is not a peptide in view of the presence of the Michael acceptor the inhibitor can conjugates through the amin acids of the inhibitor to a nanoparticle in essentially the same manner as peptide. Non-limiting examples of types of nanoparticles (NPs) are iron oxide NPs (e.g. iron oxide magnetic NPs or Amino-dextran-functionalized superparamagnetic iron oxide nanoparticles (SPION)), magnetic NPS (e.g. magnetic nanovesicles), metal NPs (e,g, gold or silver NPs), superparamagnetic spherical polymer NPs (e.g. Dynabeads or Superparamagnetic polymeric micelles (SPPM)), semiconducting NPs, mesoporous silica NP, liposomes and polymer NPs (e.g. PEG-PLL-PLGA, Poly-lactic acid, PLG/ chitosan, PLA/chitosan or polyacryl NPs).
The present invention relates in a third aspect to a pharmaceutical or diagnostic composition comprising the cathepsin inhibitor and/or the fusion construct of the invention.

The definitions and preferred embodiments of the first and second aspect of the invention apply *mutatis mutandis* to the third aspect of the invention as far as being amenable with this third aspect.

In accordance with the present invention, the terms "pharmaceutical composition" and "diagnostic composition" relate to a composition for administration to a patient, preferably a human patient. As discussed above, a diagnostic composition may also be used in vitro. The pharmaceutical composition or diagnostic composition of the invention comprises at least one inhibitor and/or the fusion construct of the invention. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The preferred form is a solution. The concentration of the inhibitor in the solution is preferably about 20 mg/ml or about 130 µM, wherein the term about is preferably ±20%, more preferably ±10%.

These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g inhibitor per day. However, a more preferred dosage might be in the range of about 0.01 mg to about 100 mg, even more preferably about 0.1 mg to about 50 mg and most preferably about 0.5 mg to about 15 mg inhibitor per day.

Furthermore, the total pharmaceutically effective amount of the inhibitor in the pharmaceutical composition administered will typically in the range of about 10 to about 150 µg per kg of body weight, such as for example about 150, 140, 130, 120, 110, 100, 90, 80, 70, 60, 50, 40, 30, 20, or 10 µg per kg of body weight. More preferably, the pharmaceutically effective amount of the inhibitor in the pharmaceutical composition will be in the range of about 6.66 to about 100 nmol of the inhibitor per kg of body weight, such as for example about 100, 90, 80, 70, 60, 50, 40, 30, 20, 10, 9, 8, 7 or 6.66 nmol of the inhibitor per kg of body weight.

For systemic administration, a therapeutically effective amount or dose can be estimated initially from *in vitro* or *ex vivo* methods. For example, a dose can be formulated in animal models to achieve a circulating concentration range that includes the IC50 as determined in cell culture. Such information can be used to more accurately determine useful doses in humans. Initial doses can also be estimated from *in vivo* data, e.g. animal models, using techniques that are well known in the art. One of ordinary skill in the art can readily optimize administration to humans based on animal data and will, of course, rely on the subject being treated, on the subject's weight, the severity of the disorder, the manner of administration.

The length of treatment needed to observe changes and the interval following treatment for responses to occur vary depending on the desired effect. The particular amounts may be determined by conventional tests which are well known to the person skilled in the art.

Pharmaceutical compositions of the invention preferably comprise a pharmaceutically acceptable carrier or excipient. By "pharmaceutically acceptable carrier or excipient" is meant a non-toxic solid, semisolid or liquid filler, diluent, encapsulating material or formulation auxiliary of any type (see also Handbook of Pharmaceutical Excipients 6ed. 2010, Published by the Pharmaceutical Press). Examples of suitable pharmaceutical carriers and excipients are well known in the art and include phosphate buffered saline solutions, water, emulsions, such as oil/water emulsions, various types of wetting agents, sterile solutions, organic solvents including DMSO etc. Compositions comprising such carriers or excipients can be formulated by well-known conventional methods. The pharmaceutical composition may be administered, for example, parenterally, such as subcutaneously, intravenously, intramuscularly, intraperitoneally, transdermally, locally or topically to the skin. The pharmaceutical composition is preferably administered parenterally, more preferably subcutaneously, and most preferably via subcutaneous injection.

The diagnostic composition of the invention is, for example, useful in the detection of an undesired physiological cathepsin levels, e.g. in different cells, tissues or another suitable sample, comprising contacting a sample with the inhibitor of the invention or the fusion construct of the invention and detecting the presence of cathepsin in the sample. Accordingly, the diagnostic composition of the invention may be used for assessing the onset or status of a disease. As defined herein below, in particular cancers and immunological disorders can be diagnosed with the help of inhibitor of the invention or the fusion construct of the invention. In this context the fusion partner of the inhibitor of the invention is preferably a fluorescent dye, a photosentisizer, a radionuclide, or a contrast agent for medical imaging. Such fusion constructs are particularly suitable for diagnostic applications.

The diagnostic composition of the invention can be administered as sole active agent or can be administered in combination with other agents, if the diagnostic composition is, for example, used to identify sites of undesired physiological cathepsin levels within a subject. In general terms the diagnostic composition of the invention is used in the diagnosis of a cathepsin-mediated disease or a cathepsin - mediated organ damage, such as cancers and immunological disorders.

The present invention relates in a fourth aspect to the cathepsin inhibitor, the fusion construct or the pharmaceutical composition of the invention for use in treating cancer or an immunological disorder.

The definitions and preferred embodiments of the first to third aspect of the invention apply *mutatis mutandis* to the fourth aspect of the invention as far as being amenable with this fourth aspect.

Cathepsins are highly expressed in various human cancers, associated with tumor metastasis (Tan et al. (2013), World J Biol Chem.; 4(4):91-101). As a group of lysosomal proteinases or endopeptidases, each member has a different function, playing different roles in distinct tumorigenic processes such as proliferation, angiogenesis, metastasis, and invasion. The cathepsin inhibitors of the invention are suitable for the treatment of cancer. Preferred examples of cancer will be described herein below.

Cathepsins are also involved in producing immune modulators by the limited proteolysis processing. For example, cathepsin B (CatB) is involved in the production of interleukin-1β (IL-1β) and TNF-α and cathepsin K (CatK) involved in toll-like receptor 9 (TLR9) activation. In addition, cathepsin S (CatS) mediates TLR signaling transduction and regulates major histocompatibility complex (MHC) class II-dependent CD4+ T-cell activation. Both the production and proteolytic activities of CatS are upregulated under conditions closely related to chronic inflammatory diseases, including periodontitis. (Yan et al. (2020), Evid Based Complement Alternat Med.; 2020:451758). The cathepsin inhibitors of the invention are therefore also suitable for the treatment of immunological disorders.

Immunological disorders are diseases or conditions caused by a dysfunction of the immune system. The expression "immunological disorder" in the context of the present invention includes autoimmune disorders that involve activity of antigen presenting cells as B-cell, macrophages, NK20 cell and T-cells. Non-limiting examples of immunological disorders are periodontitis, diabetes, cardiovascular diseases, and neurodegenerative disease, including Alzheimer disease, systemic lupus erythematosus, Sjogren's syndrome, coeliac disease and psoriasis.

In accordance with preferred embodiment of the second aspect of the invention treating cancer is an anti-cancer immunotherapy or an anti-metastatic therapy.

While it is believed that a cathepsin S inhibitor exerts its anti-cancer effect via an anti-cancer immunotherapy it is believed that a cathepsin B inhibitor exerts its anti-cancer effect via an anti-metastatic therapy.

The inhibition of tumor-associated cathepsins, especially cathepsin S (CatS), has emerged as a novel promising strategy in cancer immunotherapy. CatS is prominently expressed in tumor associated M2-type macrophages (TAM), dendritic cells (DC), and myeloid-derived suppressor cells (MDSC) of the TME (Fuchs et al. (2020), Cells; 9(9):2021). The expression "immunotherapy" includes strategies used to activate effector immune cells to increase the efficacy of the patient's own immune response against cancer cells.

Cathepsin B has been shown to directly activate MMP-1 and MMP-3, which in turn can cleave components of the ECM such as collagen, gelatin and tenascin thus facilitating the migration of tumor cells through the extracellular space (Tan et al. (2013), World J Biol Chem.; 4(4):91-101). Hence, cathepsin B helps tumors to form metastases.

In accordance with a further preferred embodiment of the second aspect of the invention the cancer is selected from the group consisting of breast cancer, ovarian cancer, endometrial cancer, vaginal cancer, vulvacancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemias.

The above cancer types are non-limiting but preferred types of cancer that can be treated with the inhibitor, the fusion protein or the composition of the invention. The list comprises the most frequent types of cancer in human, noting that lung, breast and colorectal cancer are the 3 most abundant types of cancer.

Unless otherwise defined, all technical and scientific terms used herein have the same meaning as commonly understood by one of ordinary skill in the art to which this invention belongs. In case of conflict, the patent specification including definitions, will prevail.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

This also holds true for alternatives in different claims that depend from each other. Thus, if claim 1 recites three alternatives of the same category and claim 2 recites three alternatives of a different category as recited in claim 1, and refers back to claim 1, all combinations of the alternatives as recited in claims 1 and 2 are explicitly disclosed herein.

The above considerations apply *mutatis mutandis* to all appended claims

The figures show.
**Figure 1****. A-C** FRET curve of CTSS WT (A), CTSS Y132D (B) and CTSH (C) during 180 minutes of incubation with the P3 inhibitor.
**Figure 2****.** Summary of single-site saturation mutagenesis screening results. Residual activity of CTSS measured by FRET assay, normalized to the activity observed when P3 is used to inhibit CTSS. All inhibitors were tested at 15 uM.
**Figure 3****. A** Dose-response curve of CTSS wild-type activity after 50 minutes of incubation with NPI C10. B CTSS mutated (Y132D) activity after 50 minutes of incubation with NPI C10 at the indicated concentrations.
**Figure 4****.** Summary of single-site saturation mutagenesis screening results. Residual activity of CTSB measured by FRET assay, normalized to the activity observed when P3 is used to inhibit CTSB. All inhibitors were tested at 7.5 uM.
**Figure 5****.** Dose-response curve of CTSB (top) and CTSS (bottom) activity after 50 minutes of incubation with NPI D5.
**Figure 6****. A-B** Results of internalization assay performed by incubating WSU-DLCL2 cells with the Epr-AF488 antibody for 3 hours at 37°C (A) or 4°C (B). Single-cell fluorescence was measured by flow cytometry.
**Figure 7****. A-B** CD74 accumulation after 48 hours-long anti-CD22 APeC treatment in WSU cells (A) and Raji cells (B). **C-D** CD74 accumulation after 48 hours-long anti-CD79 APeC treatment in WSU cells (C) and Raji cells (D). Single-cell fluorescence was measured by flow cytometry. CTSS inhibitor Petesicatib was used as positive control.
**Figure 8****.** Output of quenched activity-based probe (qABP) assay. The presence of a dark band corresponds to the detection of a strong activity of CTSB. Absence of a band means complete CTSB inhibition. APeC = antibody-peptide conjugate. Ab = antibody.
**Figure 9****.** Antibody conjugation strategy. The antibody first reacts with a DBCO-maleimide linker, then with the azide-modified NPI C10. Image adapted from "DBCO or azide reactive liposomes", liposomes.org.

The examples illustrate the invention.

### Example 1 - Generation of a cathepsin S inhibitor

To generate a new cathepsin S inhibitor, it was decided to start from the structure of the human protein CD74, which is a natural substrate of cathepsin S, and we computationally identified the region of the protein that is recognized and cleaved by cathepsin S. After validating the identified peptide by showing in a FRET assay that it is cleaved by cathepsin S, we synthesized multiple candidate inhibitors starting from the peptide structure, by adding a Michael acceptor at different positions in the molecule. Testing the candidates' inhibitory activity by FRET assay allowed to identify a first molecule able to bind and inhibit cathepsin S **(Structure 1).**

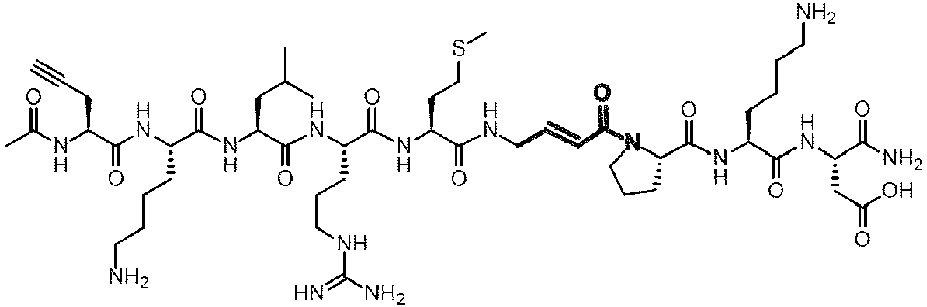

**Structure 1.** Non-natural peptide inhibitor P3. The structure of the Michael acceptor is highlighted in bold.

Then, a single-site saturation mutagenesis screen was performed to identify the amino acid changes that would improve the affinity of our inhibitor for cathepsin S. The screening allowed us to identify several inhibitors of cathepsin S more potent than our previously identified P3 **(****Figure 2****,** in the *Results* section). Based on these results and follow-up experiments, a non-natural peptide inhibitor was identified as a very potent inhibitor of cathepsin S and was named C10. Its structure is the following:

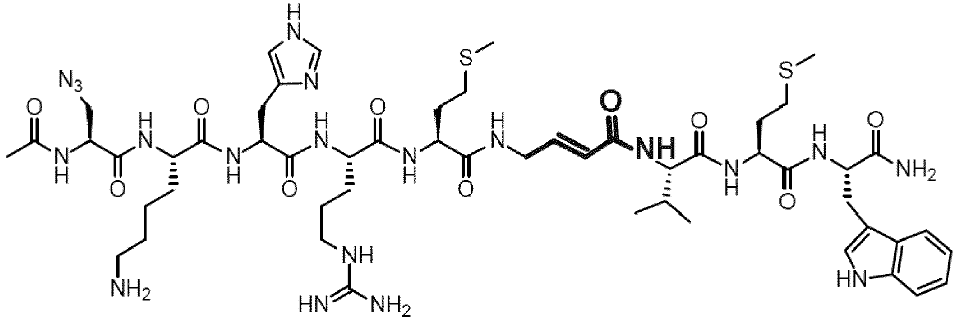

**Structure 2.** Non-natural peptide inhibitor C10. The structure of the Michael acceptor is highlighted in bold.

### Example 2 - Generation of a cathepsin B inhibitor

Then, an additional single-site saturation mutagenesis screen on cathepsin B was used to identify the amino acid changes that can be combined to obtain a strong cathepsin B inhibitor based on the cathepsin S inhibitor as obtained in Example 1. Information from both screenings was used to select amino acid changes that could improve the specificity of the inhibitor for cathepsin B or cathepsin S.

The strongest non-natural peptide inhibitor for cathepsin B that was identified with this screening and follow-up experiments was named D5. Its structure is the following:

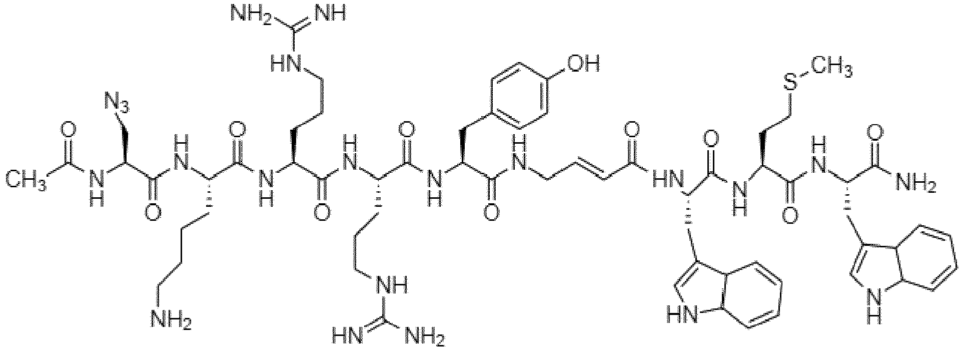

**Structure 3.** Non-natural peptide inhibitor D5. The structure of the Michael acceptor is highlighted in bold.

### Example 3 - Conjugates with therapeutic antibodies

Next, the conjugation of the non-natural peptide inhibitors as obtained in Examples 1 and 2 with the therapeutic anti-CD22 antibody Epratuzumab was optimized to generate an antibody-peptide conjugate (APeC). The APeC was tested on the WSU-DLCL2 and Raji lymphoma cell lines and the tests showed that the APeC is internalized by lymphoma cells and inhibits cathepsin S in these cells. The same conjugation strategy was then used to demonstrate the efficacy of a second APeC that can target lymphoma cells, which was obtained by conjugating the C10 inhibitor with an anti-CD79 antibody. Finally, to demonstrate that this strategy can be generalized to target other tumor types, an anti-HER2 antibody (Trastuzumab) was conjugated to our C10 inhibitor and tested CTSB inhibition in a breast cancer cell line. The general structure of our three APeCs is the following:

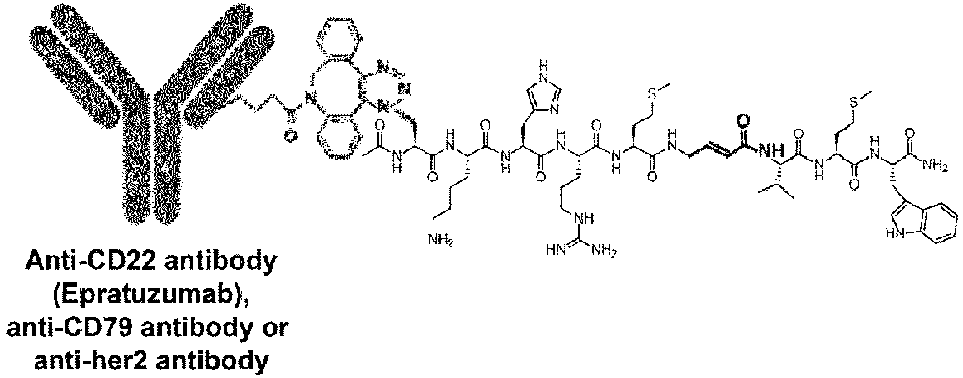

**Structure 4.** General structure of antibody-peptide conjugates carrying the C10 inhibitor of cathepsin S. The structure of the Michael acceptor is highlighted in bold.

### Example 4 - Materials and methods

**Cathepsins protein expression and purification.** For CTSS purification, the human CTSS sequence depleted of the lysosomal localization signal (amino acids 17 to 331) was amplified from pLVX plasmids expressing WT or Y132D CTSS using the following primers:
CTSS_pHLsec_fw: GCTGAAACCGGTCAGTTGCATAAAGATCCTAC (SEQ ID NO: 1)
CTSS_pHLsec_rev: GTGCTTGGTACCGATTTCTGGGTAAGAGGGAAAGC (SEQ ID NO: 2).

The PCR products were digested using Agel and Kpn1 restriction enzymes (NEB, cat #R3552S and #R3142S), then cloned in pHLsec plasmids carrying a secretion signal at the N-terminal of the MCS and a His-tag sequence at the C-terminal of the MCS. pHLsec-CTSS plasmids were transfected into HEK293 cells with PEI (polyethyleneimine) in presence of VPA (valproic acid). Proteins were purified from the supernatant using a 5 ml His-Trap FF column on an ÄKTA pure system (GE healthcare). Bound proteins were eluted using a 300 mM imidazole solution. Concentrated proteins were further purified by size exclusion chromatography on a HiLoad^{®} 16/600 Superdex^{®} 75 pg column (GE Healthcare) using PBS as mobile phase. Purified proteins were quantified by Nanodrop, diluted in a buffer containing 12.5 mM MES, 75 mM NaCl and 50% glycerol at ph 6.5 and stored at -20°C. Purified CTSB and CTSH were purchased from RndSystems.

**FRET (Fluorescence Resonance Energy Transfer) assay to test CTSS activity.** For FRET experiments, CTSS-His was diluted to 10 µg/mL in assay buffer (50 mM NaOAc, 5 mM DTT, 250 mM NaCl), at pH 4.5 and incubated at 37°C for 90 minutes to allow auto-activation. The protein was then diluted to 0.5 ng/ul in assay buffer and plated in 96-well plates (50 ul/well). As substrate, we used a peptide (KLRMKLPKP) produced by the PPCF Facility (UNIL) and diluted at 20 uM in assay buffer. The peptide synthesized by the PPCF Facility had a MCA group at the C-term and a DNP group at the N-term. The peptide was added to the activated CTSS (50 ul/well) and fluorescent emission (MCA) was measured with SpectraMax iD3 (Molecular Devices) in kinetic mode for three hours (excitation: 330 nm, emission: 390 nm).

**Single-site saturation mutagenesis screening.** We tested a library of 133 non-natural peptide inhibitors (NPls), each differing from our initial inhibitor design for only one amino acid in the structure. The peptides were synthesized by GL Biochem (Shanghai), and we tested them using the CTSS FRET assay described above. The FRET assay was miniaturized to perform the screen in a 384-well plate and each NPI was tested in duplicates. CTSS was incubated at 10 ug/ml in the activation buffer for 105 minutes to allow a complete activation of the enzyme. Then, the enzyme was diluted at 1 ug/ml and dispensed in a 396-well plate pre-loaded with the NPls using an automatic dispenser (Biotek Dispenser Multiflo). The plates were incubated for 30 minutes at room temperature and then 20 uM of substrate was added to each well. The residual CTSS activity was measured by acquiring the fluorescence signal after 90 minutes (excitation: 330 nm, emission: 390 nm). In the plate, we included untreated wells as negative control and we used E64 (150 uM) as positive control. To assess the quality of the screen, a Z'-score was calculated for the plate and its value was confirmed to be Z' > 0.6.

The saturation mutagenesis screening was used to identify stronger CTSS inhibitors bearing single amino acid changes compared to our initial inhibitor, and to select modifications that would allow to design new NPls with the potential to show even stronger activity on CTSS. 13 new NPls featuring combinations of promising amino acid modifications were synthesized and tested by FRET assay. The strongest inhibitor of cathepsin S identified through this process, which we will refer to as "C10", was selected to perform a dose-response curve by FRET assay and cellular assays to determine its efficacy in a cellular environment.

**Specificity assay.** To define whether our non-natural peptide inhibitor of cathepsin S could also inhibit the activity of other cathepsins as cathepsin B (CTSB) or cathepsin H (CTSH), we developed a FRET assay to test CTSB and CTSH activity. First, we used specific conditions to activate the enzymes. CTSB was diluted in a specific activation buffer (MES 25 mM, DTT 5 mM, pH 5) at 10 ug/ml, incubated at 37°C for 2h and then diluted to 1 ug/ml in assay buffer (MES 25 mM, pH 5) for the FRET assay. CTSH was diluted in a different activation buffer (MES 50 mM, CaCl₂ 10 mM, NaCl 150 mM, pH 6.5) at 200 ug/ml. In addition, as co-stimulatory activator we used thermolysin diluted at 100 ug/ml in activation buffer and then mixed with an equal volume of the diluted CTSH. The solution was incubated for 1h at room temperature and then we added 2 mM Phosphoramidon diluted in assay buffer (MES 50 mM, pH 6.5) and we incubated the mix for 10 minutes at room temperature. Lastly, we added an equal volume of assay buffer containing 20 mM DTT and incubated the solution for 5 minutes at room temperature. For the FRET assay, we diluted both CTSH, and CTSB at 1 ug/ml in their specific assay buffer and incubated them with a specific substrate. We used as substrate KPPKPVSD in CTSB FRET assays and KLRMKLPKP in CTSH FRET assays and we tested two doses of our P3 inhibitor (20 uM and 50 uM). Fluorescence emission was measured with SpectraMax iD3 (Molecular Devices) in kinetic mode over three hours (excitation: 330 nm, emission: 390 nm).

**Antibody conjugation.** To generate a new molecule that can be internalized by B-cells and lymphoma cells to reach cathepsin S molecules and inhibit them, our cathepsin S inhibitor (C10) was conjugated to the Epratuzumab or an anti-CD79 antibody, thereby producing an antibody-peptide conjugate (APeC). As a first step, 30-fold molar excess of TCEP was added to the solution of antibody in PBS, and the mix was incubated at room temperature for 20 minutes. Then, 20-fold molar excess of DBCO-maleimide linker molecule was added and the solution was incubated for 1 hour at room temperature to label cysteines in the antibody. Later, the excess of linker molecule was removed by running the solution through an Econo-Pac 10DG Desalting Column (Bio-Rad, cat. no. 7322010) following the manufacturer's instructions. Finally, 4-fold molar excess of our azide-C10 inhibitor was added to the solution which was incubated overnight at 4°C. To perform antibody internalization assays, Epratuzumab was conjugated with the AlexaFluor-488 azide-dye (ThermoFisher Scientific, A10266) instead of our C10 inhibitor (we will refer to the labelled antibody as "Epr-AF488"). The Epr-AF488 antibody was subsequently purified from the excess of dye with an Econo-Pac 10DG Desalting Column. To test the conjugation with an antibody targeting breast cancer cells, our C10 inhibitor was conjugated to an anti-her2 antibody (Trastuzumab) following the same protocol described above for Epratuzumab conjugation.

**Internalization assay.** To test the internalization of the conjugated Epratuzumab antibody, 2×10⁵ WSU-DLCL2 cells were incubated at 37°C for 3 hours with 100 ug/ml of Epr-AF488 in PBS. As negative control, the same number of cells was incubated with with 100 ug/ml of Epratuzumab previously incubated with AF488 dye in absence of DBCO-maleimide linker. Each experimental condition was tested in duplicates. Cells were then washed 3 times with PBS and 1 ul of anti-AF488 quencher antibody (ThermoFisher Scientific, A-11094) was added to half of the samples. Samples' fluorescence was measured by flow cytometry using Millipore Guava EasyCyte HT instrument (MilliporeSigma).

**CD74 accumulation assay for efficacy assessment.** To test the efficacy of our APeCs, 2×10⁵ WSU-DLCL2 or Raji cells were incubated for two days with 300 or 500 ug/ml of APeC, in RPMI 1640 medium (ThermoFisher Scientific) supplemented with 10% FBS and 1% Penicillin-Streptomycin (ThermoFisher Scientific, cat. no. 15140122). After the first 24h treatment, the cells were washed and resuspended in fresh APeC at the same treatment concentration. As negative controls, the same number of cells was either treated with PBS or with 300 ug/ml of the corresponding antibody previously incubated with C10 inhibitor in absence of DBCO-maleimide linker. Each treatment condition was tested in duplicates. At the end of the second day of treatment, cells were fixed and permeabilized with BD Cytofix/Cytoperm Fixation/Permeabilization Kit (BD Biosciences, cat. no. 554714) following the manufacturer's instructions. Cells were then stained with anti-human CD74 antibody (BioLegend, cat. no. 357604) and fluorescence was measured by flow cytometry using BD LSRFortessa instrument (BD Biosciences).

**Quenched activity-based probe assay to assess cellular CTSB inhibition.** To test the efficacy of our APeC for CTSB inhibition, 2×10⁵ MCF-7 cells were incubated for 16 hours with 500 ug/ml of APeC, in Gibco DMEM medium (ThermoFisher Scientific) supplemented with 10% FBS and 1% Penicillin-Streptomycin (ThermoFisher Scientific, cat. no. 15140122). As negative controls, the same number of cells was either treated with PBS or with 500 ug/ml of anti-HER2 antibody previously incubated with C10 inhibitor in absence of DBCO-maleimide linker. Each treatment condition was tested in duplicates. At the end of the 16-hours treatment, cells were incubated for 1 hour with a pan-cathepsin probe (BMV109, quenched-activity based probe). The cells were then centrifuged at 10'000 g for 1 min at RT, the supernatant was removed, and the cells were taken up in 9 µl hypotonic lysis buffer (50 mM PIPES pH 7.4, 10 mM KCI, 5 mM MgCI, 4 mM DTT, 2 mM EDTA, and 1% NP-40). The lysate was incubated on ice for 5 min, followed by centrifugation at 21'130 g for 15 min at 4°C. The supernatant was transferred, diluted with 4x sample buffer (3 µl) and the mixture was heated for 5 min at 95°C. The samples were spun down and separated by 15% SDS-PAGE. The gel was analysed by scanning in-gel fluorescence on a Typhoon Trio flat-bed laserscanner (GE Healthcare), and protein loading was confirmed by staining with Coomassie^{®} Brilliant Blue R-250 (Schmidt GmbH). When the probe binds to CTSB, a fluorescent band can be observed in the SDS-PAGE gel. Since the BMV109 probe binds to CTSB only if the enzyme is active (i.e. not inhibited), the presence of a dark band corresponds to the detection of a strong activity of CTSB, while the absence of a band means complete CTSB inhibition.

### Example 5 - Results

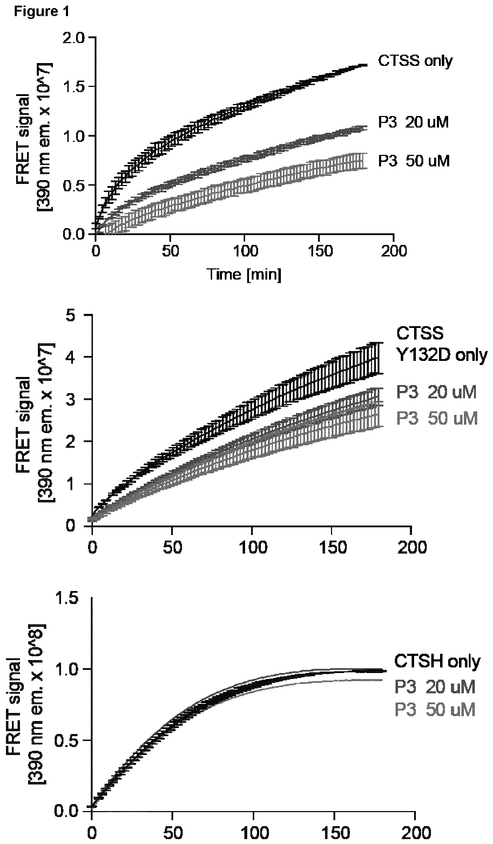

To test the inhibitory potential of the first non-natural peptide inhibitor (NPI) of cathepsin S (CTSS) which was designed, named P3, a specific FRET assay for CTSS we performed. It was observed that P3 was able to inhibit both the wild-type as well as the mutant form of CTSS at 20 uM and at 50 uM **(****Figure 1A-B****).** To test the specificity of the P3 inhibitor, a specific FRET assay for cathepsin H (CTSH) was performed. The assay showed that CTSH is not significantly inhibited by P3 at 20 uM or 50 uM **(****Figure 1C****).**

Overall, these results suggested that the P3 inhibitor selectively inhibits CTSS, without significantly affecting CTSH activity.

To screen for more potent inhibitors of cathepsin S, a single-site saturation mutagenesis screen was performed, in which 133 different NPls were tested, each differing from P3 for only one amino acid in their structure. The output of the screening is summarized in **Figure 2****.** Several of the new NPls tested had a stronger inhibitory activity on CTSS as compared to P3 (labelled as below 1.0). In particular, some promising amino acid changes were identified in positions 2 (phenylalanine and histidine), 5 (valine), 6 (leucine and methionine) and 7 (phenylalanine and tryptophan).

Besides identifying stronger CTSS inhibitors bearing single amino acid changes compared to the initial inhibitor, these results show that it is possible to design new NPls with the potential to show even stronger activity on CTSS.

After testing multiple candidates issued from the saturation mutagenesis screening, a FRET assay was performed to test in a dose-dependent manner the inhibitory activity of our most promising NPI, named C10. The dose-response curve obtained showed that our C10 NPI inhibits CTSS with an IC50 of 49.3 nM in the FRET assay which we designed **(****Figure 3A****).**

It was recently reported that 5.7% of lymphoma patients harbour a specific gain of function mutation changing the tyrosine 132 in aspartate (Y132D) or asparagine (Y132N). Hence, it was checked whether the cathepsin inhibitor that was identified in the screen (C10) is also effective on the mutated form of CTSS. This was measured through a FRET assay in which the inhibitor was incubated with CTSS Y132D. It was observed that C10 reduces the activity of CTSS Y132D of more than 50% when tested at 50 nM, and this inhibition efficiency grows to more than 90% at 500 nM **(****Figure 3B****).**

Overall, these results suggested that C10 is a potent non-natural peptide inhibitor of both the wild-type and the mutated forms of CTSS.

Since the initial NPI showed some inhibitory activity on CTSB, the same library of 133 different NPls was used to screen for cathepsin B inhibitors. The output of the screening is summarized in **Figure 4****.** Several new NPls having a stronger inhibitory activity on CTSB compared to P3 (labelled as below 1.0) were identified. In particular, it was observed that some amino acid changes could improve the activity and specificity for CTSB in positions 2 (arginine), 4 (lysine and tyrosine) and 5 (tryptophan).

After testing multiple candidates designed on the basis of the saturation mutagenesis screening output, a FRET assay was performed to test in a dose-dependent manner the inhibitory activity of the most promising NPI, named D5. The dose-response curve obtained showed that D5 NPI inhibits CTSB with an IC50 of 61.93 nM in the FRET assay as described herein **(****Figure 5****).** A similar dose-response assay was performed to test the inhibition of CTSS by D5, for which the IC50 value obtained was much higher (86.57 uM, **Figure 5****).** These results suggested that D5 is a potent and specific non-natural peptide inhibitor of CTSB.

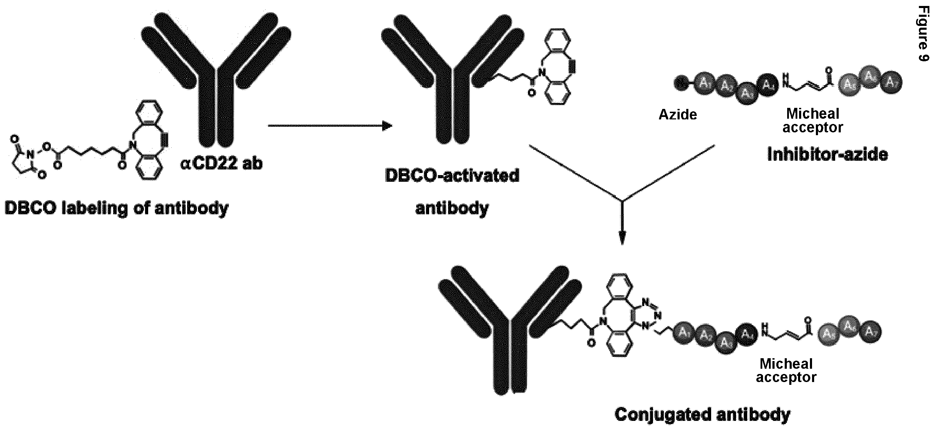

To conjugate our C10 inhibitor with the Epratuzumab antibody or the anti-CD79 antibody, a version of the NPI was used that was modified to bear an azide group at the N-term, and the antibody was labelled with a commercially available DBCO-maleimide linker, as illustrated in **Figure** 9. The final conjugation reaction occurs between the azide group and the DBCO group, and is based on copper-free click chemistry. The product of this conjugation reaction is referred to herein with the general name "antibody-peptide conjugate" (APeC). To perform antibody internalization assays, the Epratuzumab antibody was conjugated with AlexaFluor-488 azide-dye instead of our C10 inhibitor (we will refer to the labelled antibody as "Epr-AF488").

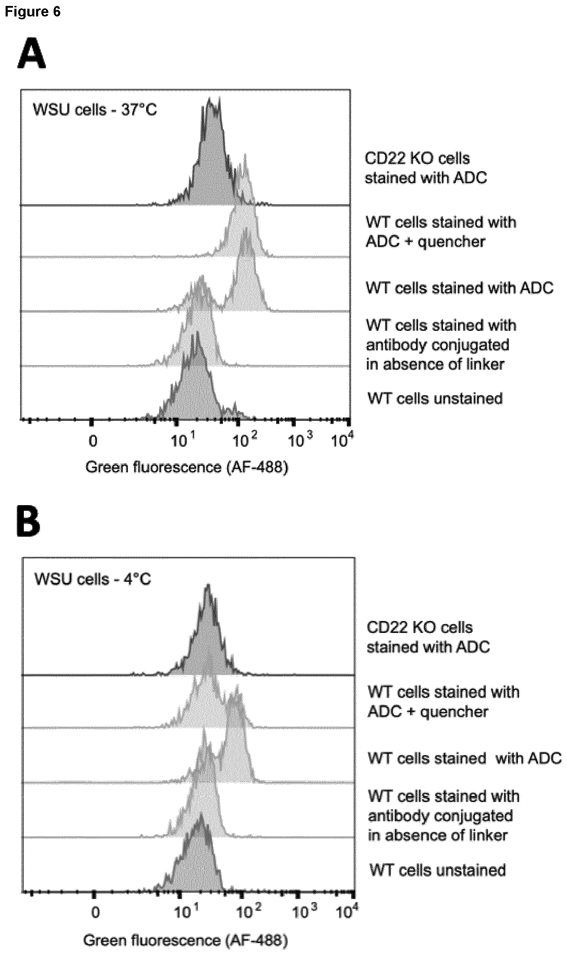

To test the binding and internalization of our APeC, an internalization assay was performed with WSU-DLCL2 cells and the Epr-AF488 antibody. The assay showed that the conjugated antibody was still able to bind lymphoma cells. Moreover, the addition of a quencher antibody allowed to assess internalization of the labelled Epratuzumab, showing that at 37°C Epr-AF488 was internalized by lymphoma cells and thus inaccessible to the quencher (**Figure 6**).

In summary, the results of this flow cytometry assay indicated that the antibody conjugation strategy generates molecules that retain the ability to bind to lymphoma cells and be internalized by them.

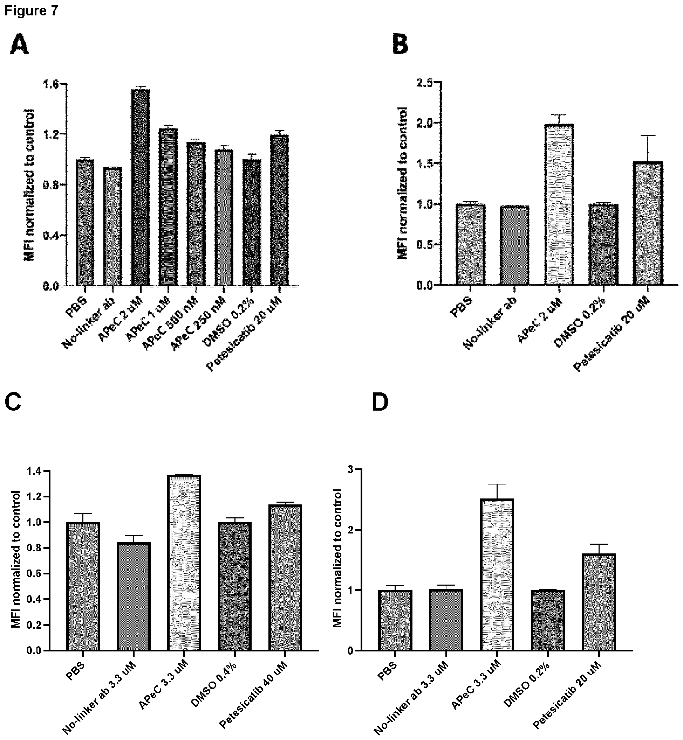

To test the efficacy of our APeCs to inhibit CTSS in lymphoma cells, a CD74 accumulation assay was performed with WSU-DLCL2 cells as well as with Raji cells. It was observed that cells treated for 48 hours with our APeCs showed greater CD74 accumulation compared to cells treated with the same amount of PBS or antibody mixed to C10 in absence of linker molecules **(****Figure 7**). Moreover, the CD74 accumulation levels measured when treating with high doses of the CTSS inhibitor Petesicatib were lower than what we obtained with lower doses of our APeCs.

Overall, these results suggested that treatment of lymphoma cells with our APeCs results in CTSS inhibition in such cells.

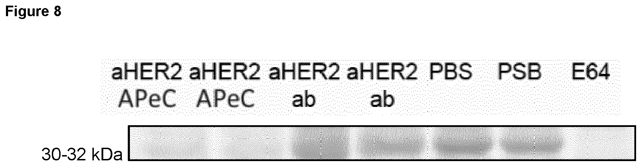

Finally, to assess if the above described strategy could be generalized to the inhibition of a different cathepsin in a different tumor type, the inhibition of CTSB in a breast cancer cell line, MCF-7, was tested through a qABP assay. It was observed that cells treated with anti-HER2 APeC for 16 hours showed lower residual CTSB activity as compared to cells treated with the same amount of PBS or antibody mixed to C10 in absence of linker molecules **(****Figure 8**). E64, a strong protease inhibitor, was used as positive control and inhibited CTSB activity as expected.

## Claims

1. A cathepsin inhibitor comprising or consisting of Formula (I)
(X₁)(X₂)(X₃)(X₄)(Y)(X₅)(X₆)(X₇) Formula (I)
wherein
(X₁) is an amino acid selected from K, L, F and M;
(X₂) is an amino acid selected from L, A, D, E, F, G, H, I, K, M, N, S, W, Y, Q and R;
(X₃) is an amino acid selected from R, A and L;
(X₄) is an amino acid selected from M, I, K, V and Y;
(X₅) is an amino acid selected from P, A, I, L, V, W and Y;
(X₆) is an amino acid selected from K, A, E, F, G, I, L, M, N, Q, S, T, V, W, Y and Q;
(X₇) is an amino acid selected from D, A, E, F, G, I, L, M, N, P, T, V, W, Y, Q and R; and
(Y) is a Michael acceptor.

2. The cathepsin inhibitor of claim 1, wherein the cathepsin inhibitor selectively inhibits cathepsin S and comprises or consists of Formula (II)
(X₁)(X₂)(X₃)(X₄)(Y)(X₅)(X₆)(X₇) Formula (II)
wherein
(X₁) is an amino acid selected from K, L, M, and is preferably K;
(X₂) is an amino acid selected from L, A, D, E, F, G, H, I, M, N, Q, S, W, Y, and is preferably F or H;
(X₃) is an amino acid selected from R and L;
(X₄) is the amino acid M;
(X₅) is an amino acid selected from P, A, I, L, V, W, and is preferably V;
(X₆) is an amino acid selected from K, A, E, F, G, I, L, M, N, Q, S, T, V, W, Y, and is preferably selected from F, L, M, Wand Y, and is most preferably L or M;
(X₇) is an amino acid selected from D, A, E, F, G, I, L, M, N, P, Q, T, V, W, Y, and is preferably selected from F, I, L, V, W and Y and is most preferably F or W.

3. The cathepsin inhibitor of claim 2, wherein the cathepsin inhibitor comprises or consists of KLRM(Y)PKD or KHRM(Y)VMW and preferably comprises or consists of KHRM(Y)VMW.

4. The cathepsin inhibitor of claim 1, wherein the cathepsin inhibitor selectively inhibits cathepsin B and comprises or consists of Formula (III)
(X₁)(X₂)(X₃)(X₄)(Y)(X₅)(X₆)(X₇) Formula (III)
wherein
(X₁) is the amino acid K or F;
(X₂) is an amino acid selected from A, H, I, K and R, and is preferably H or R;
(X₃) is an amino acid selected from A, R and L, and is preferably L;
(X₄) is an amino acid selected from I, K, V and Y, and is preferably K or Y;
(X₅) is an amino acid selected from A, I, Y, V and W, and is preferably V or W;
(X₆) is an amino acid selected from A, F, L, M, T and V, and is preferably L or M;
(X₇) is an amino acid selected from A, F, I, L, N, P, R, W and Y and is preferably F or L.

5. The cathepsin inhibitor of claim 4, wherein the cathepsin inhibitor comprises or consists of KRRY(Y)WMW.

6. The cathepsin inhibitor of any one of claims 1 to 5, wherein the Michael acceptor is an enone, a nitro group or a sulfonyl fluoride and is preferably an enone.

7. The cathepsin inhibitor of claim 6, wherein the Michael acceptor has the general formula (IV) or (V) 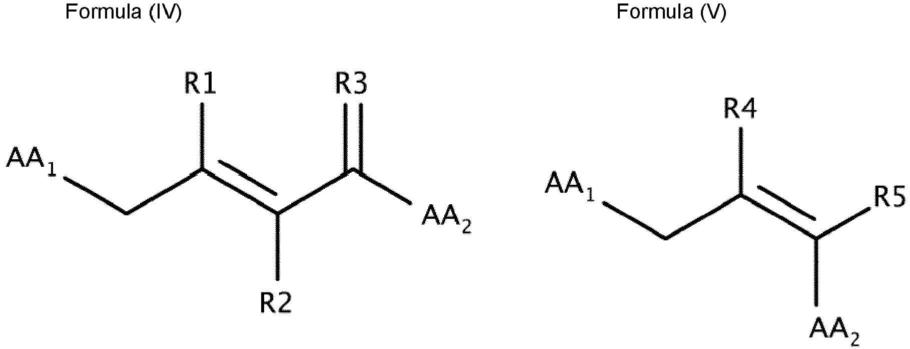 wherein
R1, R2 and R4 each independently is H or a halogen, wherein the halogen is preferably F;
R3 is chalcogen and is preferably O or S; and

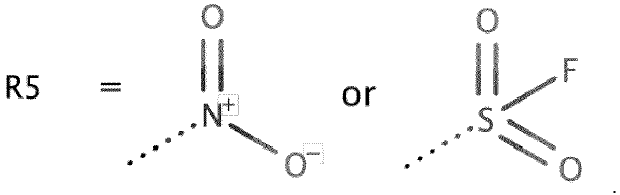

8. The cathepsin inhibitor of any one of claims 1 to 7, wherein the cathepsin inhibitor inhibits cathepsin S or B with IC₅₀ of below 200 µM, preferably below 100µM and most preferably below 75µM.

9. The cathepsin inhibitor of any one of claims 1 to 8, wherein the cathepsin inhibitor comprises at its N- or C-terminus, preferably at its C-terminus a linking moiety.

10. The cathepsin inhibitor of claim 9, wherein the linking moiety is an azide, alkyne phenol, secondary or tertiary amine, hydroxyl group, carbamate, or carbonate group, and is preferably an azide.

11. A fusion construct, wherein the cathepsin inhibitor of any one of claims 1 to 8 is fused to a heterologous compound, preferably fused to a pharmaceutically and/or diagnostically active compound.

12. The fusion construct of claim 11, wherein the heterologous compound is selected from the group consisting of:
(i) antibody or fragment thereof;
(ii) an antibody mimetic, preferably selected from the group consisting of Anticalins, Affibodies, Adnectins, DARPins, Avimers, Nanofitins, Affilinss, β-Wrapins, ADAPT, Monobodies, Raslns, FingRs, Pronectins, Centyrins, Affimers, Adhirons, Affitins, αReps, Repebodies, i-bodies, Fynomers and Kunitz domain proteins;
(ii) a cytokine, preferably cytokines selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1alpha and IL-1beta;
(iii) a toxic compound, preferably a small organic compound or polypeptide, and preferably a toxic compound selected from the group consisting of calicheamicin, neocarzinostatin, esperamicin, dynemicin, kedarcidin, maduropeptin, doxorubicin, daunorubicin, auristatin, Ricin-A chain, modeccin, truncated Pseudomonas exotoxin A, diphtheria toxin and recombinant gelonin;
(iv) a chemokine, preferably a chemokine selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, Eotaxin, Eotaxin-2, I-309, MPIF-1, 6Ckine, CTACK, MEC, Lymphotactin and Fractalkine;
(v) a fluorescent dye, preferably a component selected from a Alexa Fluor or Cy dye;
(vi) a photosensitizer, preferably bis(triethanolamine)Sn(IV) chlorine6 (SnChe6);
(vii) a pro-coagulant factor, preferably a tissue factor;
(viii) an enzyme, preferably an enzyme selected from the group consisting of carboxy-peptidases, glucuronidases and glucosidases;
(ix) a radionuclide selected either from the group of gamma-emitting isotopes, preferably 99mTc, 1231, 111In, or from the group of positron emitters, preferably 18F, 64Cu, 68Ga, 86Y, 1241, or from the group of beta-emitters, preferably 1311, 90Y, 177Lu, 67Cu, or from the group of alpha-emitters, preferably 213Bi, 211At;
(x) nanoparticles.

13. A pharmaceutical or diagnostic composition comprising the cathepsin inhibitor of any one of claims 1 to 8 and/or the fusion construct of claim 11 or 12.

14. The cathepsin inhibitor of any one of claims 1 to 9, the fusion construct of claim 11 or 12 or the pharmaceutical composition of claim 13 for use in treating cancer or an immunological disorder.

15. The cathepsin inhibitor, fusion construct or pharmaceutical composition for use of claim 14, wherein the cancer is selected from the group consisting of breast cancer, ovarian cancer, endometrial cancer, vaginal cancer, vulvacancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemias.
